# EUROPEAN PATENT SPECIFICATION

(11) **EP 3 849 629 B1**
(45) Date of publication and mention of the grant of the patent: **09.11.2022**
(21) Application number: 19769735.2
(22) Date of filing: 10.09.2019
(51) Int. Cl.: A61M 1/06

(54) **BREAST PUMP**
BRUSTPUMPE
TIRE-LAIT

(30) Priority: 13.09.2018 EP 18194238
(43) Date of publication of application: 21.07.2021
(73) Proprietor: Koninklijke Philips N.V., 5656 AG Eindhoven (NL)
(72) Inventor: BOURQUIN, Yannyk Parulian Julian, 5656 AE Eindhoven (NL); BROCKHUIS, Lili-Marjan, 5656 AE Eindhoven (NL); SEGAAR, Lucja Elzbieta, 5656 AE Eindhoven (NL); HAEX, Nicole Petronella Martien, 5656 AE Eindhoven (NL)
(74) Representative: Philips Intellectual Property & Standards
(86) International application number: PCT/EP2019/074013
(87) International publication number: WO 2020/053159

(56) References cited:
- US-A1- 2008 177 224
- US-B1- 8 052 635

## Description

### FIELD OF THE INVENTION

The present invention relates to a breast pump and a non-therapeutic method of operating a breast pump.

### BACKGROUND OF THE INVENTION

Breast pumps are well known and are used by breast feeding women to express milk from their breast and to collect the milk in a bottle such that the milk can be fed to a baby.

Typically, an electrical breast pump comprises a vacuum pump connected to a funnel via a tube. The breast of a user is placed into the funnel, the vacuum pump is activated and milk is expressed into a bottle.

US2008/177224 A1 discloses a programmable electric breast pump system that includes a vacuum pump pneumatically coupled to breast cups and a vacuum relief valve bypassing the vacuum pump. It also includes a controller that receives command input from a vacuum level selector and a vacuum rate selector, and feedback from a pressure sensor measuring vacuum in the breast cups. In response, the controller cycles the relief valve between minimum and maximum vacuum set points to create a periodic vacuum pulse in the breast cups. By adjusting the vacuum min/max levels and vacuum rate, a user may change the strength of the pulse after let-down, or synchronize the frequency of the pulse with the natural refractory time of a lactating breast.

US 2017/0087285 A1 discloses a breast pump with a codified container for receiving expressed breast milk. A computing device receives an image of the expressed milk in the container and the codification allows a software to recognize the size and type of the container as well as the scale and orientation and to translate the image into an accurate milk volume. The milk data can be processed and analyzed to produce a feedback regarding the pumping session.

As the extraction of milk by means of a breast pump can be time consuming and may be considered uncomfortable by the user, it is desirable to have a breast pump that can efficiently extract milk from the breast of the user.

### SUMMARY OF THE INVENTION

It is therefore an object of the invention to provide a breast pump which can efficiently express milk from the breast of a user in particular with an increased amount of expressed milk in a reduced period of time.

According to an aspect of the invention, a breast pump comprises a vacuum pump, a controller and a breast fullness estimator which is able to estimate the fullness of the breast (the amount of milk inside the breast). The breast fullness estimator estimates the breast fullness based on at least one of a duration of a current milk expression session, a time of day of the milk expression session and information from previous milk expression sessions. The controller selects one of the different vacuum profile settings based on the breast fullness estimation of the breast fullness estimator. Hence, the efficiency of the breast pump is increased as the operation of the vacuum pump is adapted to the estimated breast fullness.

According to an embodiment, if a milk extraction session is started, then the time of day is logged. Furthermore, the milk extraction history is analyzed and based on the milk extraction history and the time of day, a specific vacuum pump profile is chosen when the milk extraction session has started. The vacuum pump is then operated based on the selected vacuum profile setting. Based on the analysis of the breast fullness estimator, namely the amount of milk in the breast at specific times of day during the day, the vacuum pump can be controlled with the various vacuum settings.

For example, if based on the previous milk extraction history, it is known that the amount of milk in the breast in the evening is low, then a first vacuum profile can be selected for the vacuum pump parameters. On the other hand, if a milk expression session is to be started in the morning or at night when typically, the amount of milk inside the breast is high, then a different vacuum profile setting can be selected with different vacuum parameters for the vacuum pump. Accordingly, the breast pump can be operated more efficiently based on the estimation of breast fullness, i.e. the amount of milk in the breast at a specific time of day.

According to an embodiment, the breast pump comprises a memory for logging information regarding previous milk extraction sessions to provide milk expression history data.

According to an embodiment, the vacuum profile setting includes a vacuum level, a vacuum rate, an atmosphere rate, a dwell in time and/or a dwell out time.

According to an embodiment of the invention, the breast pump device comprises a sensor configured to estimate a breast fullness at a start of a milk expression session or during the milk expression session.

According to an embodiment, the controller changes vacuum profile settings during a milk ejection session based on a breast fullness during the milk expression session.

According to an aspect of the invention, a non-therapeutic method of operating a breast pump which comprises a vacuum pump which can be operated at different vacuum profile settings is provided. A fullness of a breast is estimated based on at least one of a duration of a current milk expression session, a time of day of the milk expression session and information from previous milk expression sessions. An operation of the vacuum pump is controlled based on the different vacuum profile settings. One of the different vacuum profile setting is selected based on the breast fullness estimation from the breast fullness estimation step.

According to an embodiment a vacuum pump of the breast pump is controlled based on an estimated fullness of a breast of a user.

According to an aspect of the invention a computer program for operating a breast pump is provided. The computer program comprises program code means for causing a breast pump to carry out the steps of the method of operating a breast pump, when the computer program is run on a computer controlling the breast pump.

### BRIEF DESCRIPTION OF THE DRAWINGS

In the following drawings:
Fig. 1 shows a schematic block diagram of a breast pump according to an embodiment of the invention,
Fig. 2 shows a schematic flow chart of an operation of a breast pump according to an embodiment of the invention,
Fig. 3 shows a basic representation of a vacuum profile of the breast pump according to an embodiment of the invention, and
Fig. 4 shows a graph depicting an influence of vacuum profile parameter to milk extraction.

### DETAILED DESCRIPTION OF EMBODIMENTS

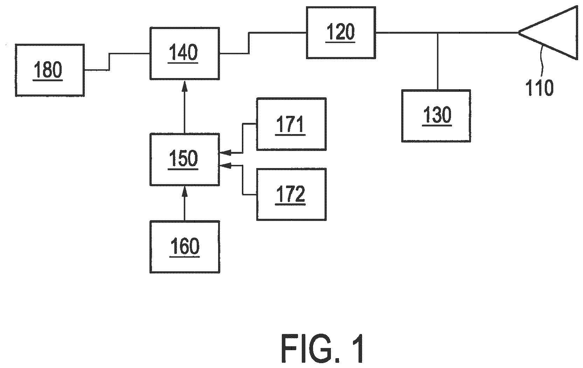

Fig. 1 shows a schematic block diagram of a breast pump according to an embodiment of the invention. The breast pump 100 comprises at least one funnel 110 for receiving a breast of a user, a vacuum pump 120 for creating a vacuum in order to enable a milk expression, a container 130 into which the expressed milk can flow and a controller 140 for controlling an operation of the vacuum pump 120. The breast pump 100 also comprises a breast fullness estimator 150 which estimates the fullness of a breast. In other words, the breast fullness estimator 150 is used to estimate an amount of milk in a breast of a user. The breast pump 100 may optionally comprise a memory 160 for logging milk extraction information of previous milk extraction sessions to build up a milk expression history. The breast pump 100 may also comprise a timer 171 and a clock 172. The information from the clock 172 and from the timer 171 can be used by the breast fullness estimator 150 to estimate the amount of milk in the breast, i.e. this information is used to estimate the breast fullness.

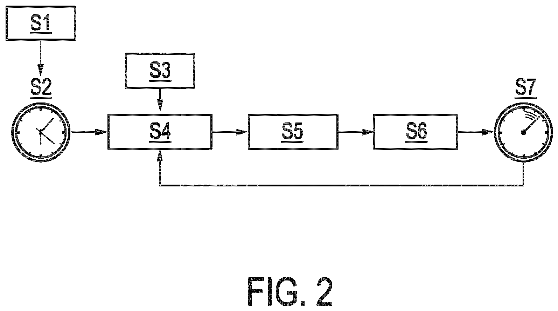

Fig. 2 shows a schematic flow chart of an operation of a breast pump according to an embodiment of the invention. The breast pump according to this embodiment corresponds to the breast pump according to Fig. 1. In step S1, the power of the breast pump 100 is switched on. In step S2, the time of day is forwarded to the breast fullness estimator. In step S3, the breast fullness estimator 150 extracts information from the memory 160 containing information regarding previous milk extraction sessions. It should be noted that step S2 can be performed before or after step S3. In other words, in step S3, information from previous milk extraction sections (milk expression history) are extracted from a memory 160. In Step S4, the breast fullness estimator 150 estimates a fullness of the breast based on the information from the clock 172 (i.e. the time of day when the milk expression history is started) and the extracted information (milk expression history) from the memory 160. In step S5, a vacuum profile is selected for the vacuum pump 120 based on the analysis of the breast fullness estimator 150. In step S6, the milk extraction session is started with the selected vacuum profile setting such that the vacuum pump 120 is operated based on the selected vacuum profile setting. In step S7, a timer is started.

The vacuum profile setting as set in step S5 may be constant during the milk expression session but may also vary during the milk expression session.

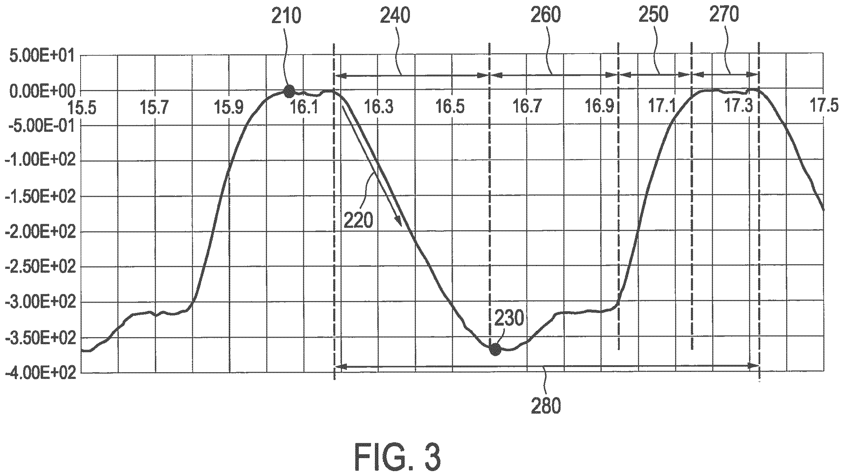

Fig. 3 shows a basic representation of a vacuum profile of the breast pump according to an embodiment of the invention. In Fig. 3, different parameters 200 of the vacuum during the milk extraction session are depicted over time. In particular, a baseline vacuum 210, a vacuum rate 220, a maximum vacuum 230, a time to vacuum 240, a time to atmosphere 250, a dwell in time 260, a dwell out time 270 as well as a cycle time 280 are depicted. The cycle time 280 can be approximately one second. Optionally, the maximum vacuum 230 can be approximately -350 mbar.

Typically, a full breast will result in a higher milk flow than an empty breast. A full breast may influence the pressure exerted during the milk ejection reflex MER. According to the invention, the vacuum profile settings may influence the milk extraction at different milk ejection reflex pressures or according to the breast fullness. The breast fullness may vary during the time of day. Typically, a breast is fuller during the night and the morning than in the afternoon or evening. Also, the breast fullness may change depending whether the baby is only fed during the day or is also fed during the night.

It should also be noted that at the start of a milk extraction session, the amount of milk in the breast is typically high, i.e. in other words, the breast is typically full at the start of a milk extraction session. As milk is expressed from the breast, the breast is typically empty at the end of the milk extraction session. Accordingly, the pressure related to the milk ejection reflex is also reduced during the milk extraction session as more milk is already extracted from the breast. As an example, based on previous milk extraction sessions, the breast fullness of a user may be average during the morning, during day, but low in the evening and high during the night. This is in particular the case if milk extraction sessions are also performed during night. On the other hand, if milk extraction sessions are not performed during the night, then the breast fullness may be high in the morning, at average during the day and low in the evening.

According to an embodiment of the invention, at least three different vacuum profile settings may be used, namely a first vacuum profile setting if the breast fullness is estimated as high, a second vacuum profile setting if the breast fullness is estimated as average and a third vacuum profile setting if the breast fullness is estimated as low.

According to an embodiment of the invention, a sensor can be used to estimate the breast fullness, in particular at the start of the milk extraction session. Such a sensor may be an impedance sensor, an inductance sensor or a nipple stretching sensor or a breast stiffness sensor.

The sensor can be used to estimate the breast fullness. The sensor needs to be in contact with the breast. It can be e.g. in the funnel or it can be an external module. In case of the impedance sensor, the sensor will typically measure the impedance of the breast containing the milk, the obtained measurement indicates the presence of milk in the breast as the presence of milk influence the measurement. The sensor can be used in combination with the breast fullness estimator according to the embodiments of the invention to increase the accuracy of the estimator. Potentially, the estimator could also be only based on the sensor measurement.

As described with reference to Fig. 2, a timer 171 can be started at the beginning of a milk expression session. According to an embodiment of the invention, 50% of the milk in the breast can be extracted after a first time interval (e.g. 4,4 minutes) and 80% of the milk inside the breast can be expressed after a second time interval (e.g. 6,9 minutes). Hence, during a milk expression session, three different settings for the vacuum parameters may be used, namely a first setting if more than 50% of milk is still inside the breast, i.e. until the first time interval, a second setting until 20% of milk is still inside the breast, i.e. until a second time interval, and a third setting if less than 20% is inside the breast, i.e. after the second time interval. The selection of these different parameters may be based on the timer and previously determined threshold values of breast fullness.

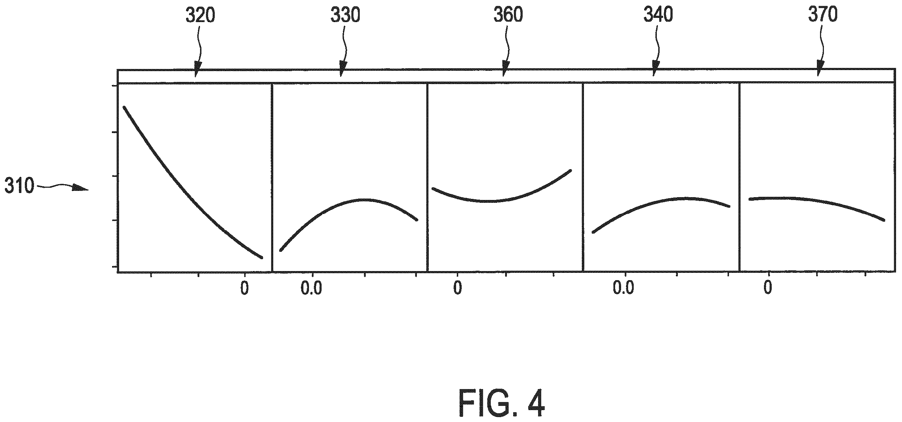

Fig. 4 shows a graph depicting an influence of vacuum profile parameter to milk extraction. In Fig. 4, the influence of the different vacuum profile parameters on the milk extraction are depicted. In Fig. 4, the weight of the expressed milk 310 is depicted in relation to the vacuum 320 [mbar], the vacuum rate 330 [mbar/ms], atmosphere rate 340 [mbar/ms], the dwell in time 360 [ms] and the dwell out time 370 [ms]. In order to reduce an irritation of the breasts, one or more pauses can be made during a session.

Accordingly, the first setting may relate to a higher vacuum level. The second setting may correspond to a typical vacuum setting and the third setting may have a longer dwell in time and a shorter dwell out time. For example, the dwell out time according to the third setting may be twice shorter than dwell in time. The same applies for the dwell out time.

The same may apply to the settings which may change during a single milk ejection session.

Other variations of the disclosed embodiment can be understood and effected by those skilled in the art in practicing the claimed invention from a study of the drawings, the disclosure and the appended claims.

In the claims, the word "comprising" does not exclude other elements or steps and in the indefinite article "a" or "an" does not exclude a plurality.

A single unit or device may fulfil the functions of several items recited in the claims. The mere fact that certain measures are recited in mutual different dependent claims does not indicate that a combination of these measurements cannot be used to advantage. A computer program may be stored/distributed on a suitable medium such as an optical storage medium or a solid state medium, supplied together with or as a part of other hardware, but may also be distributed in other forms such as via the internet or other wired or wireless telecommunication systems.

## Claims

1. Breast pump (100), comprising
a vacuum pump (120) which is configured to be operated at different vacuum profile settings,
a controller (140) configured to control an operation of the vacuum pump (120) based on the different vacuum profile settings, and
a breast fullness estimator (150) configured to estimate a fullness of a breast based on at least one of:
(i) a duration of a current milk expression session; and
(ii) a time of day of the milk expression session and information from previous milk expression sessions,
wherein the controller (140) is configured to select one of the different vacuum profile settings based on the breast fullness estimation of the breast fullness estimator (150).

2. Breast pump (100) according to claim 1, wherein option (i) involves the duration of a current milk expression session and previously determined threshold values of breast fullness.

3. Breast pump (100) according to claim 1 or claim 2, further comprising a memory (160) which is configured to log information regarding previous milk extraction sessions.

4. Breast pump (100) according to claim 1 or claim 2, wherein the vacuum profile setting includes a vacuum level (210), a vacuum rate, an atmosphere rate (250), a dwell in time (260) and/or a dwell out time (270).

5. Breast pump (100) according to claim 1 or claim 2, further comprising a sensor (180) configured to estimate a breast fullness at a start of a milk expression session or during the milk expression session.

6. Breast pump (100) according to claim 1 or claim 2, wherein the controller (140) is configured to change vacuum profile settings during a milk ejection session based on a breast fullness during the milk expression session.

7. A non-therapeutic method of operating a breast pump (100) which comprises a vacuum pump (120) which can be operated at different vacuum profile settings, comprising the steps of:
estimating a fullness of a breast based on at least one of:
(i) a duration of a current milk expression session; and
(ii) a time of day of the milk expression session and information from previous milk expression sessions,
controlling an operation of the vacuum pump (120) based on the different vacuum profile settings, and
selecting one of the different vacuum profile setting based on the breast fullness estimation from the breast fullness estimation step.

8. Non-therapeutic method of claim 7, wherein option (i) involves the duration of a current milk expression session and previously determined threshold values of breast fullness.

9. A computer program for operating a breast pump, the computer program comprising program code means configured to cause a breast pump as defined in claim 1 to carry out the steps of the method defined in claim 7, when the computer program is run on a computer controlling the breast pump.

## Patentansprüche

1. Brustpumpe (100), umfassend
eine Vakuumpumpe (120), die für den Betrieb mit unterschiedlichen Vakuumprofil-Einstellungen betrieben werden kann, eine Steuerung (140), die so konfiguriert ist, dass sie den Betrieb der Vakuumpumpe (120) auf der Grundlage der verschiedenen Vakuumprofileinstellungen steuert, und
einen Brustfülle-Schätzer (150), der so konfiguriert ist, dass er die Fülle einer Brust einschätzt auf Grundlage von mindestens einem von:
(i) einer Dauer einer aktuellen Milchentnahmesitzung; und
(ii) einer Tageszeit der Milchentnahmesitzung und Informationen aus früheren Milchentnahmesitzungen, wobei die Steuerung (140) so konfiguriert ist, dass sie eine der verschiedenen Vakuumprofileinstellungen auf der Grundlage der Brustfülle-Schätzung des Brustfülle-Schätzers (150) auswählt.

2. Brustpumpe (100) nach Anspruch 1, wobei die Option (i) die Dauer einer aktuellen Milchentnahmesitzung und zuvor bestimmte Schwellenwerte der Brustfülle umfasst.

3. Brustpumpe (100) nach Anspruch 1 oder Anspruch 2, die ferner einen Speicher (160) umfasst, der so konfiguriert ist, dass er Informationen über frühere Milchentnahmesitzungen aufzeichnet.

4. Brustpumpe (100) nach Anspruch 1 oder Anspruch 2, wobei die Vakuumprofileinstellung ein Vakuumniveau (210), eine Vakuumrate, eine Atmosphärenrate (250), eine Verweilzeit (260) und/oder eine Verweilzeit (270) umfasst.

5. Brustpumpe (100) nach Anspruch 1 oder Anspruch 2, die ferner einen Sensor (180) umfasst, der so konfiguriert ist, dass er die Brustfülle zu Beginn einer Milchentnahmesitzung oder während der Milchentnahmesitzung schätzt.

6. Brustpumpe (100) nach Anspruch 1 oder Anspruch 2, wobei die Steuerung (140) so konfiguriert ist, dass sie die Einstellungen des Vakuumprofils während einer Milchentnahmesitzung auf der Grundlage einer Brustfülle während der Milchentnahmesitzung ändert.

7. Nicht-therapeutisches Verfahren zum Betreiben einer Brustpumpe (100), die eine Vakuumpumpe (120) umfasst, die mit unterschiedlichen Vakuumprofileinstellungen betrieben werden kann, mit den Schritten:
Abschätzen der Fülle einer Brust auf der Grundlage von mindestens einem der folgenden Punkte:
(i) einer Dauer einer aktuellen Milchentnahmesitzung; und
(ii) einer Tageszeit der Milchentnahmesitzung und Informationen aus früheren Milchentnahmesitzungen, Steuern eines Betriebs der Vakuumpumpe (120) auf der Grundlage der verschiedenen Vakuumprofileinstellungen, und
Auswählen einer der verschiedenen Vakuumprofileinstellungen auf der Grundlage der Schätzung der Brustfülle aus dem Schritt der Schätzung der Brustfülle.

8. Nichttherapeutisches Verfahren nach Anspruch 7, wobei die Option (i) die Dauer einer aktuellen Milchentnahmesitzung und zuvor bestimmte Schwellenwerte der Brustfülle umfasst.

9. Computerprogramm zum Betreiben einer Brustpumpe, wobei das Computerprogramm Programmcodemittel umfasst, die so konfiguriert sind, dass sie bewirken, dass eine Brustpumpe nach Anspruch 1 die Schritte des in Anspruch 7 definierten Verfahrens ausführt, wenn das Computerprogramm auf einem Computer ausgeführt wird, der die Brustpumpe steuert.

## Revendications

1. Tire-lait (100), comprenant
une pompe à vide (120) qui est configurée pour être actionnée à différents réglages de profil de vide,
un contrôleur (140) configuré pour commander un fonctionnement de la pompe à vide (120) sur la base des différents réglages de profil de vide, et
un estimateur de plénitude mammaire (150) configuré pour estimer une plénitude d'un sein
sur la base d'au moins l'un des éléments suivants:
(i) une durée d'une session d'expression du lait en cours; et
(ii) une heure de la journée de la session d'expression du lait et des informations provenant de sessions d'expression du lait précédentes, dans lequel le contrôleur (140) est configuré pour sélectionner l'un des différents réglages de profil de vide sur la base de l'estimation de la plénitude mammaire de l'estimateur de plénitude mammaire (150).

2. Tire-lait (100) selon la revendication 1, dans lequel l'option (i) implique la durée d'une session d'expression du lait en cours et des valeurs seuils de plénitude mammaire déterminées précédemment.

3. Tire-lait (100) selon la revendication 1 ou la revendication 2, comprenant en outre une mémoire (160) qui est configurée pour enregistrer des informations concernant les sessions d'extraction de lait précédentes.

4. Tire-lait (100) selon la revendication 1 ou la revendication 2, dans lequel le réglage du profil de vide comprend un niveau de vide (210), un taux de vide, un taux d'atmosphère (250), un temps d'entrée (260) et/ou un temps de sortie (270).

5. Tire-lait (100) selon la revendication 1 ou la revendication 2, comprenant en outre un capteur (180) configuré pour estimer une plénitude mammaire au début d'une session d'expression du lait ou pendant la session d'expression du lait.

6. Tire-lait (100) selon la revendication 1 ou la revendication 2, dans lequel le contrôleur (140) est configuré pour changer les réglages du profil de vide pendant une session d'éjection du lait sur la base d'une plénitude mammaire pendant la session d'expression du lait.

7. Procédé non thérapeutique d'utilisation d'un tire-lait (100) qui comprend une pompe à vide (120) qui peut être utilisée à différents réglages de profil de vide, comprenant les étapes consistant à:
estimer une plénitude d'un sein sur la base d'au moins l'un de:
(i) une durée d'une session d'expression de lait en cours; et
(ii) une heure de la journée de la session d'expression du lait et des informations provenant de sessions d'expression du lait précédentes,
commander un fonctionnement de la pompe à vide (120) sur la base des différents réglages de profil de vide, et
sélectionner l'un des différents réglages de profil de vide sur la base de l'estimation de la plénitude mammaire à partir de l'étape d'estimation de la plénitude mammaire.

8. Procédé non thérapeutique selon la revendication 7, dans lequel l'option (i) implique la durée d'une session d'expression du lait en cours et des valeurs seuils de plénitude mammaire déterminées précédemment.

9. Programme d'ordinateur pour faire fonctionner un tire-lait, le programme d'ordinateur comprenant des moyens de code de programme configurés pour amener un tire-lait tel que défini dans la revendication 1 à effectuer les étapes du procédé défini dans la revendication 7, lorsque le programme d'ordinateur est exécuté sur un ordinateur commandant le tire-lait.
